# EUROPÄISCHE PATENTANMELDUNG

(11) **EP 4 616 809 A2**
(43) Veröffentlichungstag der Anmeldung: **17.09.2025**
(21) Anmeldenummer: 25152893.1
(22) Anmeldetag: 20.01.2025
(51) Int. Cl.: A61B 6/10, A61B 6/00, A61B 6/58

(54) **SENSORISCHER HAUTSCHUTZ**

(30) Priorität: 15.03.2024 DE 102024000861
(71) Anmelder: Ziehm Imaging GmbH, 90471 Nürnberg (DE)
(72) Erfinder: Mronz, Markus, 90452 Eckental (DE); Hauser, Ewald, 91227 Leinburg (DE); Sander, Thomas, 90453 Nürnberg (DE)
(74) Vertreter: Wittmann, Günther

(57) **Zusammenfassung**

Ein medizinisches Bildgebungssystem, vorzugsweise ein Röntgengerät, besonders bevorzugt ein C-Bogen, das dazu konfiguriert ist, die Sicherheit von Patienten während eines bildgebenden Verfahrens zu verbessern, wobei das System umfasst:
a. ein C-Bogen-Röntgengerät zur Erzeugung von Röntgenstrahlung, das in der Lage ist, in verschiedene Positionen bewegt zu werden, um unterschiedliche Ansichten des Patienten zu ermöglichen;
b. eine Liege oder Patientenauflage, die dazu konfiguriert ist, den Patienten während des bildgebenden Verfahrens zu positionieren;
c. einen Abstandssensor, der dazu konfiguriert ist, den Abstand zwischen dem C-Bogen-Röntgengerät und/oder Generator und/oder C-Bogen-Gehäuse und/oder dem Patienten und/oder der Liege zu messen;
d. eine Dosisregelungseinheit, die dazu konfiguriert ist, die Strahlungsdosis des Röntgenstrahls basierend auf den gemessenen Abständen zwischen dem C-Bogen-Röntgengerät (Fokuspunkt) und dem Patienten zu regeln; und
e. ein Hautschutzsystem, das dazu konfiguriert ist, in kritischen Situationen die Strahlungsdosis zu unterbrechen und/oder zu minimieren und gleichzeitig eine ausreichende Bildqualität aufrechtzuerhalten, indem es die Strahlungsintensität in Echtzeit anpasst, um die Strahlenexposition der Patientenhaut zu begrenzen.

## Beschreibung

Röntgenverfahren bzw. Röntgengeräte werden seit Jahrzehnten verwendet, um medizinische Bilder zu erstellen, indem Röntgenstrahlen durch den Körper eines Patienten geleitet werden, unabhängig davon, ob es sich um eine lebende Person oder ein Tier handelt. Trotz des enormen medizinischen Nutzens ist eine solche ionisierende Strahlung nicht ohne Risiken für die Gesundheit des Patienten. Daher ist der Einsatz von Röntgenstrahlen bei lebenden Patienten gesetzlich streng geregelt. Insbesondere gibt es maximal zulässige Grenzwerte für die Rate der Röntgenstrahlendosis, die die Körperoberfläche des Patienten erreicht. Je näher sich der Strahler (Fokuspunkt der Röntgenröhre) am Patienten befindet, um so größer wird die Dosisbelastung. Aus diesem Grund wurde ein Mindestabstand vom Strahler, Fokuspunkt zur Haut des Patienten, eingeführt. Beispielsweise sei hier erwähnt, dass der 21st Code of Federal Regulations des U.S. Department of Health and Human Services in DHHS 21 CFR 1020.32e den sogenannten Maximum Input Exposure Factor für die erlaubte Hauteintrittsdosis festlegt. Die Verwendung von Röntgengeräten in der medizinischen Diagnostik und anderen Bereichen erfordert also eine effektive Kontrolle der Strahlung, um das Risiko von Gesundheitsschäden für Patienten und medizinisches Personal zu minimieren. Röntgengeräte mit einem mechanischen, abnehmbaren Hautschutztubus, der den Mindestabstand zum Fokuspunkt vergrößert, sind bekannt. Die vorliegende Erfindung bietet eine Lösung für diese Herausforderung durch den Einsatz von Sensoren zur Messung und Überwachung der Mindestabstände als auch zur Anpassung und/oder Abschaltung der Dosisleistung. Darüber hinaus eignet sie sich auch zur gleichzeitigen Kollisionsüberwachung.

Beim sogenannten Untertisch-Röntgenplatz wird der Röntgengenerator unter dem Patiententisch platziert. Der Patiententisch ist höhenverstellbar, d.h. der Abstand zwischen dem auf dem Tisch bzw. der Tischplatte liegenden Patienten und dem Röntgengenerator ist einstellbar. Der mit dem Patienten arbeitende Arzt kann den Tisch in eine ergonomische Arbeitsposition bringen. Die gesetzlich geforderten Mindestabstände zur Patientenhaut müssen dabei eingehalten werden.

Es ist bekannt, dass der Röntgengenerator durch Ansteuerung dauerhaft unterhalb seines maximalen Leistungsbereichs betrieben wird, also mit dem Produkt aus Röhrenstrom und Beschleunigungsspannung das geringer ist als die Röntgenquelle tatsächlich maximal emittieren kann. Es gilt generell, dass der Abstand vom Detektor zum Patienten so nah wie möglich sein sollte. Sollte dies jedoch nicht möglich sein, helfen erfindungstechnisch Sensoren bei der Anpassung der Dosis bis zur Abschaltung des Röntgenstrahlers falls der Abstand vom Patienten zum Fokuspunkt der Röhre sich verringert oder der Mindestabstand unterschritten wird. Weiterhin ist es erfindungstechnisch vorgesehen zusätzlich ein Warnsignal in akustischer, haptischer oder optischer Form auszugeben.

Die Reduzierung der Strahlungsleistung führt jedoch zu einer Abnahme der Anzahl der erzeugten Röntgenquanten bei gleichzeitiger Zunahme des Quantenrauschens. Somit sind alle Kompromisse zwischen Röntgenbildqualität, Strahlensicherheit und Ergonomie des Gesamtsystems bekannt. Die Aufgabe der vorliegenden Erfindung besteht darin, mit einem geeigneten Verfahren sicherzustellen, dass die gesetzlich vorgeschriebenen Sicherheitsabstände eingehalten und die Dosisregelung (ALARA) sowie Ergonomie verbessert werden.

ALARA = As low as reasonably achieveable.

Die Dosisrate wird im Stand der Technik automatisch angepasst. Beispielsweise können Ärzte während einer Operation automatisch auf qualitativ hochwertigere Röntgenbilder zugreifen, indem sie einfach den Behandlungstisch anheben, um Detektor-nah zu arbeiten.

Aus dem Dokument DE 1317696 U ist eine Röntgenkugel mit einem nichtabnehmbaren Dentaltubus bekannt, der als Zielvorrichtung für die Ausrichtung des Zentralstrahls verwendbar ist.

Aus dem Dokument DE 4447856 C2 ist ein Röntgenstrahler bekannt, an dessen Gehäuse parallel zum Zentralstrahl angeordnete, stabförmige, nicht abnehmbare Abstandshalter vorgesehen sind, welche einmal dem Schutz eines Röntgenstrahlenkollimators vor Stößen und zum anderen der Sicherstellung eines minimalen Fokus-Haut-Abstandes, beispielsweise zur Erfüllung von FDA-Vorgaben (US Food and Drug Administration) dienen.

Aus dem Dokument DE 3781171 T2 ist ein mobiler C-Bogen für militärische Einsätze (Philips BV 25 T) bekannt, der zur besseren Transportierbarkeit werkzeuglos in mehrere Baugruppen zerlegbar ist. Aus dem Operation Manual zu dem Gerät, englisch aus dem Jahr 2020 (http://hdl.handle.net/20.500.12091/1430, Quelle: https://www.usa.philips.com/healthcare , abgerufen am 27.04.2023) und aus der Bedienungsanleitung Philips Röntgengerät, Feld, leicht, BV 25 T, deutsch mit Erscheinungsjahr 1989 des gescannten Druckexemplars, (abgerufen am 27.04.2023) ist in den Fig. 22 und 23 und dem dazu gehörigen Beschreibungstext bekannt, dass verschiedene Tuben (für Fluoroskopie und Dental) zusammen mit einer Halteplatte entfernbar und austauschbar sind. Es wird offenbart, dass das Röntgengerät ohne aufgesetzten Tubus nicht betreibbar ist.

Aus dem Dokument DE 29520926 U1 ist ein in eine Röhrengehäusehälfte integrierter und nicht aus dem Strahlungsfeld entfernbarer Röhrentubus bekannt, in dem ein Strahlungsbegrenzungsteil lösbar gehalten ist.

Aus dem Dokument DE 60128722 T2 ist ein mobiler C-Bogen mit einem Röhrentubus zur Gewährleistung eines Sicherheitsabstandes vorgesehen; die Möglichkeit der Entfernbarkeit des Röhrentubus ist nicht offenbart.

Die Röntgengeräte gemäß vorliegender Erfindung umfassen eine Röntgenröhre zur Erzeugung von Röntgenstrahlen und einen Detektor zur Aufnahme der Strahlung. Zusätzlich sind Sensoren in das Gerät integriert, um die Strahlungsintensität sowie den Abstand zur Röntgenquelle zu überwachen und/oder mögliche Kollisionen zu detektieren. Die Sensoren können verschiedene Arten der Detektion umfassen, die für Röntgenanlagen geeignet sind.

Die erfassten Daten werden von einer Steuer- und/oder Recheneinheit analysiert, die in der Lage ist, den Abstand zum Objekt in Echtzeit zu bewerten. Basierend auf den Ergebnissen werden automatisch Anpassungen am Röntgengerät vorgenommen, um die Strahlungsbelastung zu begrenzen und/oder ganz abzuschalten. Dies kann beispielsweise die Anpassung der Röhrenspannung, des Röhrenstroms, die Pulsrate und/oder anderer Parameter umfassen, um die Strahlung zu reduzieren oder zu eliminieren. Zusätzlich kann die Bewegung des Tisches und/oder des C-Bogens angepasst werden, um den entsprechenden Abstand zu verringern bzw. zu erhöhen. Des Weiteren werden mögliche Kollisionen detektiert bzw. verhindert.

So ermöglicht die Steuer- und/oder Recheneinheit eine adaptive Anpassung des Röntgengeräts, um die Strahlenbelastung zu reduzieren und/oder zu verhindern sowie Kollisionen zu verhindern und/oder zu vermeiden, was das Risiko von Gesundheitsschäden für Patienten und medizinisches Personal minimiert. Weiterhin trägt sie zur Verbesserung der Bildqualität bei, indem sie eine optimale Strahlungsdosis für die Aufnahme von Bildern gewährleistet.

Zur weiteren Beschreibung der Erfindung wird auf die Darstellungsmöglichkeiten der Figuren verwiesen.

Ein Objekt kann ein Tisch mit oder ohne Steuerung sein, der Patient selbst oder Teile von ihm, beides oder andere im Bewegungsbereich befindlichen Teile. Es kann sich auch um Roboterassistierte Systeme handeln.

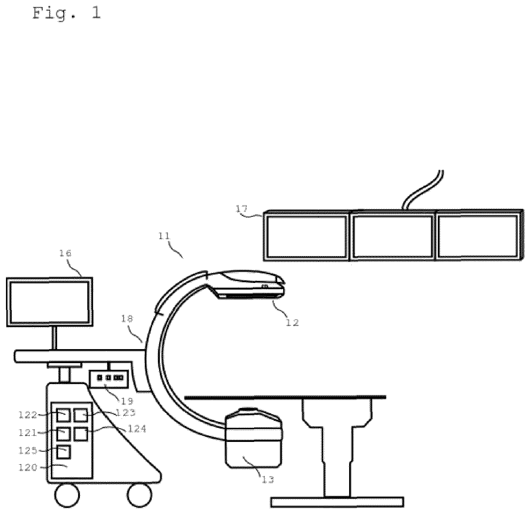

In Fig. 1 ist eine erfindungsgemäße Vorrichtung in Ausgestaltung eines mobilen C-Bogens 11 schematisch dargestellt, welcher für die Umsetzung des erfindungsgemäßen Verfahrens vorgesehen ist.

Der C-Bogen 11 trägt an seinem einen Ende einen Röntgengenerator 13, und an seinem anderen Ende, dem Röntgengenerator 13 gegenüberliegend, einen Röntgenbilddetektor 12, beispielsweise kann es sich hierbei um einen Flachdetektor oder einen Bildverstärker handeln. Der C-Bogen 11 ist in mehreren Achsen im Raum motorisch gesteuert verstellbar, wobei die Achsen Sensoren zur Erfassung des Maßes der Verstellung aufweisen.

Ferner beinhaltet die Vorrichtung eine Bildverarbeitungseinheit 121, eine Speichereinheit 122, einer Steuerungseinheit 123, sowie eine Netzwerkschnittstelle 124. Mittels der Netzwerkschnittstelle 124 können Daten, beispielsweise Bilddatensätze und Resultate des erfindungsgemäßen Verfahrens, in einem Netzwerk verteilt bzw. zur Verfügung gestellt werden.

Die Bildverarbeitungseinheit 121 umfasst eine Steuerungseinheit 122, auf dieser können die für das erfindungsgemäße Verfahren verwendeten zwei- oder dreidimensionalen Bilddatensätze gespeichert bzw. geladen werden. Diese Bilddatensätze können entweder von einem Server geladen oder mittels des C-Bogen 11 vor oder während einer Intervention aufgenommen werden. Ferner umfasst die Speichereinheit Instruktionen, die für die Ausführung des erfindungsgemäßen Verfahrens mittels einer Recheneinheit verwendet werden.

Ferner kann die Vorrichtung eine GUI 19 beinhalten, mit einer Bildausgabeeinheit (16, 17) und einer Eingabeeinheit 19, mit welchen für die Bildverarbeitungseinheit 121 entsprechende Einstellungen in entsprechenden Organprogrammen vorgenommen werden können.

Figurenbeschreibung 2: Abstandsmesssystem mit kapazitivem Prinzip Das Abstandsmesssystem basiert auf dem kapazitiven Prinzip und dient der Messung des Abstands zwischen zwei Objekten. Es handelt sich um eine nichtkontaktbasierte Methode, die auf der Erfassung von elektrischen Kapazitätsänderungen beruht.

Das Abstandsmesssystem mit kapazitivem Prinzip findet Anwendung in verschiedenen Bereichen wie der Robotik, der Automatisierungstechnik, der Fahrzeugtechnik und der Messtechnik. Es ermöglicht präzise Abstandsmessungen und trägt zur Verbesserung von Sicherheit, Genauigkeit und Effizienz in verschiedenen industriellen und medizinischen Anwendungen bei.

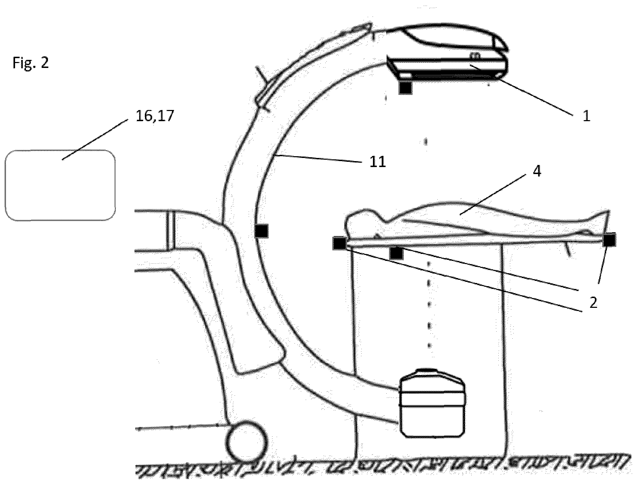

Die Figur 2 zeigt eine schematische Darstellung des Abstandsmesssystems. Ein kapazitiver Sensor basiert auf Veränderung der elektrischen Kapazität eines Kondensators bzw. Kondensatorsystems. Bei Annäherung eines leitfähigen Objektes wird das elektrische Feld des Sensors verändert was wiederum zu einer Kapazitätsänderung führt, die gemessen werden kann. Der Sensor wird in der Nähe der zu messenden Objekte positioniert, vorzugsweise am Generator- oder Detektorgehäuse. Die durch die Annäherung verursachte Kapazitätsänderung wird an eine Auswerteeinheit 123 weitergeleitet, die die gemessenen Werte analysiert und den Abstand zwischen dem Objekt und dem Sensor berechnet. Beispielhaft kann es ein einziger umlaufender Sensor sein oder auch mehrere verschiedene Sensoren.

Das kapazitive Abstandsmesssystem ist für Wasser und Metalle, daher auch für die Anwendung am Menschen besonders geeignet und bietet mehrere Vorteile gegenüber anderen Alternativen. Durch das kapazitive Prinzip kann das Abstandsmesssystem eine hohe Genauigkeit und Wiederholbarkeit bieten, da es unabhängig von Umgebungseinflüssen wie Licht, Temperatur oder Verschmutzung ist. Die umständliche Handhabung eines mechanischen Abstandstubus entfällt ebenfalls. Zudem verkleinert ein mechanischer Abstandstubus die Maulweite des C-Bogens was wiederum zu unnötigen mechanischen Kollisionen bei der Positionierung des C-Bogens führen kann auch wenn nicht gestrahlt wird. Eine Lagerung für den Abstandstubus ist ebenfalls nicht notwendig.

Im Vergleich zum Ultraschall ist ein kapazitiver Sensor nicht von Schallreflexionen abhängig und kann daher auch in Umgebungen mit starken akustischen Interferenzen eingesetzt werden. Im Vergleich zu optischen Systemen ist es weniger empfindlich gegenüber z. B. schwierigen Lichtverhältnissen. Im Vergleich zu induktiven Systemen ist es weniger anfällig für magnetische Störungen. Im Vergleich zu Radar bietet es eine höhere räumliche Auflösung und eignet sich daher für präzise Messungen in engen Bereichen.

Alle aufgeführten Alternativen sind jedoch genauso als Sensor vorstellbar.

Es kann nur ein Sensor sein, alle anderen gezeigten sind optional.

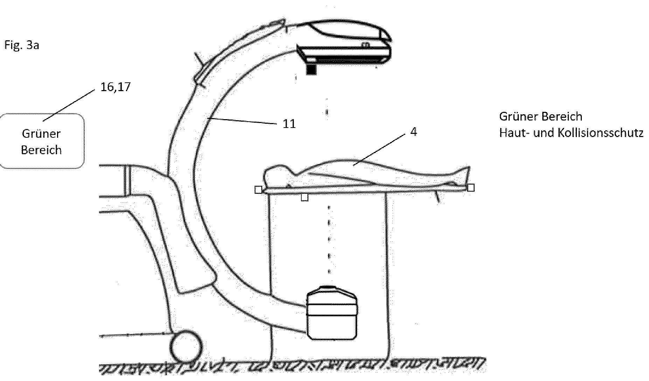

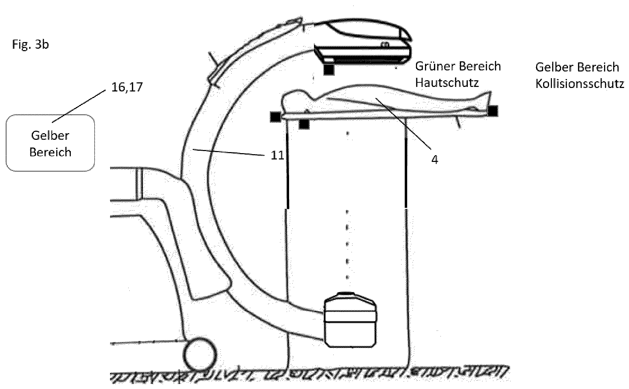

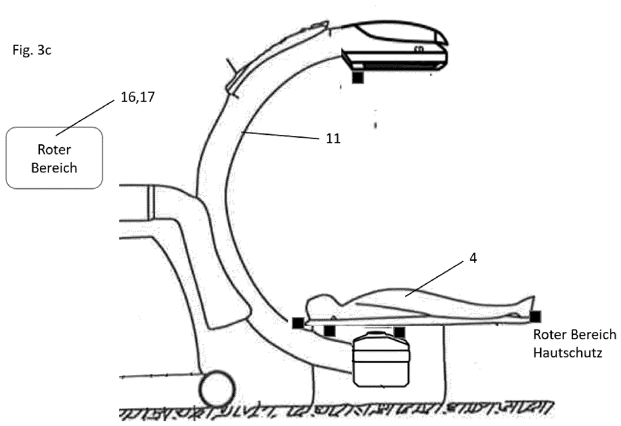

Figurenbeschreibung 3: Sensorischer Kollisionsschutz mit Reaktionsmechanismen Die Figur 3 zeigt ein System zur sensorischen Kollisionserkennung und die möglichen Reaktionen auf eine erkannte Kollision. Das System besteht aus einem oder mehreren Sensoren, einer Steuereinheit 123 und Aktuatoren zur Steuerung der Bewegung eines Objekts, wie hier eines medizinischen Gerätes, hier speziell eines Röntgengerätes und weiter bevorzugt eines C-Bogens 11.

Die Sensoren sind sinnvoll strategisch um das Objekt herum angeordnet und erfassen seine Umgebung. In der Abbildung sind verschiedene Sensortypen dargestellt, wie zum Beispiel Ultraschallsensoren, Infrarotsensoren, taktile Sensoren oder Kameras. Jeder Sensor erfasst Informationen über die Entfernung, Hindernisse oder potenzielle Kollisionen in seinem Wahrnehmungsbereich.

Der oder die Sensoren können optional an anderen sinnvollen Positionen angebracht sein, wie zum Beispiel im oder am Flatpanelgehäuse, im oder am C-Bogen direkt oder im oder am Generatorgehäuse.

Die von den Sensoren erfassten Daten werden an die Steuereinheit 123 weitergeleitet, die die Informationen analysiert und eine Entscheidung über die notwendigen Reaktionen trifft. In der Abbildung ist die Steuereinheit 123 als zentrale Einheit dargestellt, die die Sensorinformationen verarbeitet und die Bewegung des Objekts steuert.

Bei Erkennung einer Kollision oder eines nahen Hindernisses kann die Steuereinheit verschiedene Reaktionen auslösen. In der Abbildung sind zwei mögliche Reaktionsmechanismen dargestellt: das Verlangsamen der Bewegung und das vollständige Stoppen der Bewegung.

Im Fall des Verlangsamens wird die Geschwindigkeit der Achsenbewegung des Röntgengerätes und/oder des zu untersuchenden Objekts reduziert, um eine Kollision zu verhindern, indem man die Achsenbewegung stoppt und/oder die Achsenbewegung anpasst um das Hindernis zu umfahren. Dies kann beispielsweise durch eine schrittweise Verringerung der Antriebskraft und/oder durch eine Änderung der Steuerungsparameter erfolgen. Dadurch wird rechtzeitig eine Kollision verhindert.

Im Fall des vollständigen Stopps wird die Bewegung des Objekts sofort unterbrochen. Die Aktuatoren der Achsenbewegung und/oder des Objekts, wie beispielsweise Bremsen oder Antriebssysteme, werden angesteuert, um eine sofortige Stilllegung der Bewegung zu gewährleisten.

Die genaue Reaktion hängt von den spezifischen Anforderungen und dem Anwendungsbereich ab. In einigen Fällen kann es auch möglich sein, alternative Maßnahmen wie eine Änderung der Fahrtrichtung und/oder das Ausweichen um das Hindernis herum zu ergreifen.

Es wird zudem verhindert, dass der gesetzlich geforderte Mindestabstand vom Strahler zum Hauteintrittspunkt unterschritten wird, wenn gestrahlt wird.

Das sensorische Kollisionsschutzsystem mit den Reaktionsmechanismen bietet einen wirksamen Schutz vor Kollisionen und ermöglicht eine sichere Interaktion des Röntgengerätes mit seiner Umgebung. Es wird im Stand der Technik in verschiedensten Bereichen eingesetzt, darunter Robotik, Fahrzeugsicherheit, automatisierte Produktionslinien und anderen Anwendungen, bei denen die Vermeidung von Kollisionen und die Sicherheit von großer Bedeutung sind.

Die Figur 3 zeigt weiterhin eine Darstellung des Systems, das Abstandsmessung, Kollisionsschutz, Dosisregelung und Hautschutz an einem Röntgengerät 11 gewährleistet, wobei die Position des C-Bogens 11 und/oder der Liege 3 berücksichtigt wird. Das System verwendet verschiedene Sensoren und Technologien, um die genaue Positionierung und Ausrichtung des C-Bogens und/oder der Liege während der Untersuchung sicherzustellen.

Abstandsmessung: Das System verwendet verschiedene Methoden zur Abstandsmessung, darunter die Überwachung der C-Bogen Positionen über geeignete Encoder, kapazitiven Sensoren, externe oder interne Kameras (wie Navigationskamera oder ähnliches) und Muster-/Bilderkennung. Diese Technologien ermöglichen es dem System, den genauen Abstand zwischen dem Röntgengerät 11 und dem Patienten, der C-Bogen Position und/oder der Liege 3 zu ermitteln.

Kollisionsschutz: Durch die kontinuierliche Überwachung der Abstände und Positionen zum Objekt kann das System potenzielle Kollisionen erkennen. Dies wird durch die Integration von kapazitiven Sensoren und/oder Lage- und/oder Beschleunigungssensoren ermöglicht. Bei Erkennung einer möglichen Kollision werden entsprechende Maßnahmen ergriffen, um die Bewegung zu verlangsamen und/oder zu stoppen und/oder das Hindernis/Objekt zu umfahren und so Verletzungen oder Schäden zu vermeiden.

Dosisregelung: Das System passt die Strahlendosis basierend auf den erfassten Informationen an. Dabei berücksichtigt es sowohl die Position des C-Bogens 11 als auch die Position des Objekts 3. Durch die Verwendung einer GUI 19 können zusätzliche Informationen wie die Beschaffenheit (Material und/oder Dicke) des Objekts 3 eingegeben werden, um die Dosisregelung weiter zu optimieren. Die Dosis kann je nach gewähltem Organprogramm oder Betriebsmodus unterschiedlich ausgewertet und angepasst werden.

Unter Organprogramm und Betriebsmodus kann man folgende Bereiche definieren: Bereich "Standarddosis": Dieser Bereich repräsentiert die Standarddosis, die für die meisten Röntgenuntersuchungen verwendet wird. Hier wird die Strahlung mit einer vordefinierten Dosis abgegeben, die als sicher und angemessen für die allgemeinen Untersuchungen angesehen wird.

Bereich "Dosis herunterregeln": In bestimmten Fällen, in denen eine geringere Strahlendosis ausreicht oder aus medizinischen Gründen erforderlich ist, wird die Strahlung in diesem Bereich heruntergeregelt. Das System passt die Strahlungsparameter an, um die Dosis zu reduzieren und den Patienten einer geringeren Strahlendosis auszusetzen.

Bereich "Abhängig vom gewählten Organprogramm": Dieser Bereich zeigt eine personalisierte Dosisregelung, die auf die zu untersuchende Zielregion und unterschiedlichen Applikationen (z.B. Organ, Knochen, Gefäße etc.) zugeschnitten ist. Das System berücksichtigt die spezifischen Anforderungen jeder Zielregion und die gewünschte Bildqualität, um die optimale Dosis zu liefern. Zusätzlich kann die Dosis in Abhängigkeit vom Abstand zum Objekt gemäß des ALARA Prinzips angepasst werden.

Durch die visuelle Darstellung des Dosisstatus ermöglicht das Display 16,17 dem Bediener eine Echtzeitüberwachung und Kontrolle der Dosisregelung. Es bietet eine intuitive Schnittstelle, um die Strahlendosis an die spezifischen Bedürfnisse jedes Patienten und jeder Untersuchung anzupassen, um sowohl die Bildqualität als auch die Sicherheit zu optimieren.

Bereich "Strahlung anpassen": Der Hautschutz wird ebenfalls in das System integriert. Basierend auf den Abstandsmessungen und der Positionierung des C-Bogens 11 und/oder der Liege 3 kann das System den Hautschutz bewerten und angemessene Maßnahmen ergreifen, um die Strahlenbelastung auf die Haut zu minimieren. Dies kann die Anpassung der Strahlungsdosis oder andere geeignete Maßnahmen umfassen. Es wird eine Warnung ausgegeben, die auf das Erreichen des Mindestabstandes hinweist und die Strahlendosis automatisch reduziert. Bereich "Strahlung unterbrechen": In Situationen, während gestrahlt wird und dabei der Mindestabstand durch Bewegung des Objektes und/oder des C-Bogens unterschritten wird, wird die Strahlung in diesem Bereich vollständig unterbrochen, um eine potenzielle Hautschädigung zu vermeiden.

Bereich "Strahlung nicht auslösen": Dieser Bereich wird angezeigt, wenn der Mindestabstand zwischen der Röntgenquelle und der Haut des Patienten unterschritten ist. Das System reagiert, indem es die Strahlung nicht auslöst, um die Haut weiter zu schützen. Gleichzeitig wird eine Warnung angezeigt, um den Bediener auf die kritische Unterschreitung des Mindestabstands hinzuweisen. Bereich "Warnung": Dieser Bereich wird angezeigt, wenn der Abstand zwischen der Röntgenquelle und der Haut des Patienten zwar noch sicher ist, aber sich dem kritischen Punkt nähert. Das System gibt dem Bediener eine Warnung, um auf die erhöhte Gefahr hinzuweisen und ihn zur Vorsicht zu mahnen.

Bereich "Regelung der motorischen Bewegungen": Dieser Bereich zeigt an, dass das System die motorischen Bewegungen des Röntgengeräts reguliert, um einen zu engen Nachlauf zwischen der Röntgenquelle und der Haut des Patienten zu vermeiden. Die Regelung erfolgt, um sicherzustellen, dass der Abstand angemessen bleibt und eine ausreichende Hautschutzdosis gewährleistet wird.

Die vorgenannten Varianten sind beispielhaft zu sehen und dienen der näheren Veranschaulichung. Alle anderen möglichen Darstellungs- und Ausführungsformen sind ebenfalls denkbar.

Die Figur 3 zeigt in einer weiteren möglichen Ausführungsform die Darstellung eines C-Bogens 11 mit integrierter Positionsermittlung der Achsen und verschiedenen Sensoren, die eine präzise Achsenpositionierung und Ausrichtung mit Hilfe von geeigneten Wegerfassungssystemen ermöglichen können. Das System kann mehrere Technologien zur genauen Bestimmung der Achsenposition und Ausrichtung des C-Bogens 11 während der medizinischen Untersuchungen innerhalb des Raumes und/oder in Relation zum Objekt nutzen.

Externe oder interne Kameras: Das System kann externe oder interne Kameras wie eine Navigationskamera verwenden, um die Position des C-Bogens 11 zu erfassen. Diese Kameras nehmen kontinuierlich Bilder oder Videos auf und analysieren diese mit Hilfe von Muster- oder Bilderkennungsalgorithmen, um die genaue Position des C-Bogens 11 zu bestimmen.

Eingegebene Informationen über die GUI 19: Zusätzlich zur Positionsermittlung könnten über die grafische Benutzeroberfläche (GUI) 19 des Systems auch Informationen über die Beschaffenheit (Material und/oder Dicke und/oder Patienteninformationen) des Objekts eingegeben werden. Diese Informationen werden verwendet, um die Auswertung der C-Bogen 11 Position zu ergänzen und die optimale Konfiguration des Systems zu gewährleisten.

Lage- und/oder Beschleunigungssensoren: Das System kann mit Lage- und/oder Beschleunigungssensoren ausgestattet sein, um Bewegungen und Ausrichtung des C-Bogens 11 zu überwachen. Diese Sensoren erfassen kontinuierlich die Lage, Position, Beschleunigung und Bewegungen des C-Bogens 11 und ermöglichen eine präzise Bestimmung der Lage, Geschwindigkeit, Bewegungsrichtung und Ausrichtung.

Schnittstelle zur Patientenliege: Das System kann zusätzlich auch über eine Schnittstelle mit der Patientenliege 3 verbunden sein, um Informationen über deren Position und Lage zu erhalten. Dies ermöglicht eine enge Integration zwischen der Position des C-Bogens 11 und der Position des Patienten auf der Liege 3. Mit den gekoppelten Systemen aus C-Bogen und Patientenliege, lassen sich die aktivierten (C-Bogen und OP-Tisch) Achsen koordiniert bewegen, ansteuern und abstimmen um z.B. Kollisionen zu vermeiden und/oder den Patienten in eine optimale Position für die Bilderfassung zu bringen. Weiterhin lässt sich das System so optimal ergonomisch für die vorgesehenen Behandlungen und/oder Arbeitsabläufe des Anwenders einstellen und steuern.

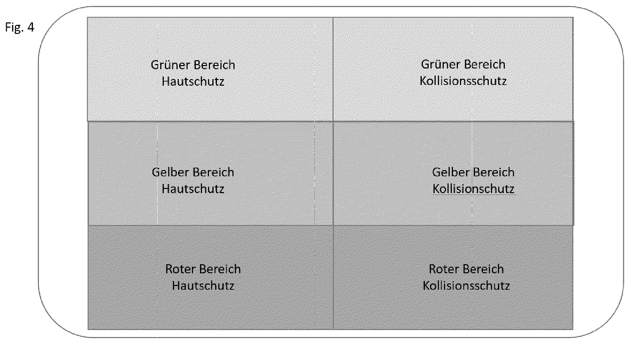

Die Figur 4 zeigt die Darstellung des sensorischen Kollisionsschutzsystems und der Dosisanpassung am Display 16,17 einer Steuereinheit 123. Das Display 16,17 dient der visuellen Anzeige des Abstands zum Objekt und/oder potenziellen Kollisionspunkten und/oder der Dosisanpassung und/oder Ausgabe von Warnungen/Hinweisen. Die Darstellung erfolgt erfindungsgemäß mithilfe z.B. einer Farbcodierung, um den Status des Kollisionsschutzes deutlich zu kennzeichnen.

Die Darstellung kann aber auch akustisch, visuell, haptisch oder anders erfolgen. Folgende gezeigte Darstellung ist beispielhaft für ein mögliches Szenario und kann auch anders farblich und technisch umgesetzt werden.

Auf dem Display wird die Farbcodierung verwendet, um den Grad des Risikos darzustellen und die entsprechenden Reaktionen anzuzeigen. Es können mehrere Bereiche angezeigt werden oder nur der jeweilige kritische/relevante.

Grüner Bereich: Der grüne Bereich repräsentiert einen ausreichenden Abstand für sowohl den Hautschutz als auch den Kollisionsschutz. Hier besteht keine unmittelbare Gefahr, und das System arbeitet im Normalbetrieb, ohne Änderungen an der Strahlungsdosis oder der Ansteuerung der Achsenmotoren vorzunehmen.

Gelber Bereich I: Der gelbe Bereich I zeigt einen kritischen, aber noch erlaubten Abstand für den Hautschutz. Hier ist der Abstand so gering, dass der Hautschutz nicht mehr als unkritisch gesehen werden kann. Das System reagiert, indem es die Strahlungsdosis herunterregelt, um eine angemessene Sicherheit nach dem ALARA Prinzip zu gewährleisten.

Gelber Bereich II: Der gelbe Bereich II kennzeichnet einen kritischen Abstand für die Kollision. Hier ist der Abstand zum Objekt zu gering, um eine Kollision zu vermeiden. Das System reagiert, indem es die entsprechende Bewegung verlangsamt, und somit die Gefahr einer Kollision mit einem Hindernis und/oder Objekt minimiert.

Roter Bereich I: Der rote Bereich I weist auf einen zu geringen Abstand für den Hautschutz hin. Hier ist der kritische Abstand so gering, dass selbst bei verlangsamter Bewegung, der gesetzliche Mindestabstand zur Haut unterschritten wird, in diesem Zustand ist die Strahlungserzeugung verboten. Das System reagiert, indem es die Strahlungserzeugung unterbricht, und den Bediener informiert und gleichzeitig auffordert, das Gerät in einen gelben oder grünen Bereich zu verfahren. Erst dann ist eine Strahlung wieder möglich.

Roter Bereich II: Der rote Bereich II kennzeichnet einen zu geringen Abstand für die Kollisionsvermeidung. Hier besteht eine hohe Wahrscheinlichkeit einer Kollision, selbst bei verlangsamter Bewegung. Das System reagiert, indem es die Achsenbewegung und/oder die Bewegung des Objektes vollständig stoppt, um eine Kollision zu verhindern. Auch hier wird die Bewegung nur in die sicheren Bereiche erlaubt.

Durch die visuelle Darstellung des Abstands und der entsprechenden Farbcodierung ermöglicht das Display eine intuitive Überwachung und Steuerung des Haut- und Kollisionsschutzsystems. Der Bediener kann den Status auf einen Blick erkennen und gegebenenfalls geeignete Maßnahmen zum Patientenschutz zu ergreifen.

Weiterhin ist ein manueller Eingriff, nach Sicherheitsbestätigung möglich, um im eigenen Ermessen des Arztes, das System zu überstimmen.

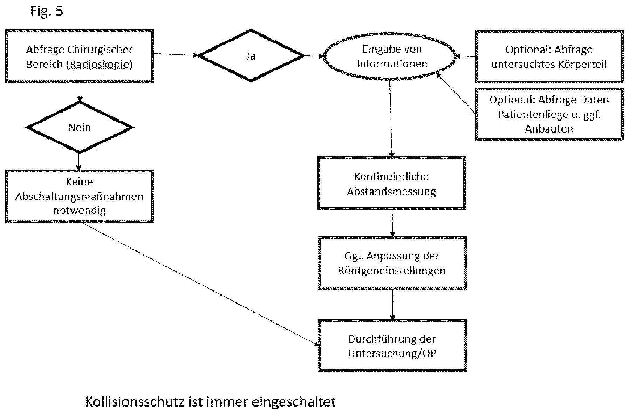

Die Figur 5 beschreibt einen beispielhaften Workflow des Systems.

Folgende Abfragen sind beispielhaft und können über eine Benutzeroberfläche GUI 19 oder andere Eingabemethoden erfolgen. Sich wiederholende Abläufe können auch fest im System hinterlegt werden.

Abfrage des chirurgischen Bereichs: Das System überprüft zu Beginn, ob der C-Bogen 11 in einer chirurgischen Anwendung eingesetzt wird. Falls dies der Fall ist, kann der Hautschutzabstand, gemäß den normativen Angaben, angepasst werden.

Optionale Erfassung besonderer Gegebenheiten: Optionale Eingabe Beschaffenheit der Liege, wie Dicke, Material oder zusätzliche Anbauten an der Liege und/oder der zu untersuchenden Körperregion.

Abstandsmessung mit Sensoren: Das System verwendet Sensoren zur präzisen Abstandsmessung zwischen dem C-Bogen 11 und dem untersuchten Körperteil. Die Sensoren erfassen den aktuellen Abstand und liefern Echtzeitdaten für die weitere Verarbeitung.

Berücksichtigung der Patientenliege 3 zur Kollisionsvermeidung und Hautschutz: Abhängig von der Positionierung des C-Bogens 11 (Generator unterhalb oder seitlich von der Patientenliege) wird der zu untersuchende Bereich erfasst und gegebenenfalls berücksichtigt das System die bekannte Dicke der Liege 3. Dies stellt sicher, dass der Abstand korrekt für die Berechnungen und Einstellungen verwendet wird.

Anpassung der Röntgeneinstellungen: Basierend auf dem gemessenen Abstand und den eingegebenen Informationen zur Beschaffenheit und Dicke des untersuchten Körperteils und/oder der Patientenliege (Objekts) 3, passt das System die Röntgeneinstellungen an. Dies umfasst beispielsweise die Strahlungsintensität und Belichtungszeit, um eine akzeptable Bildqualität bei gleichzeitiger Minimierung der Strahlendosis zu gewährleisten oder ggfs. einen Abbruch der Strahlung bei Unterschreitung des Mindestabstands.

Durchführung der Untersuchung: Nach Anpassung der Röntgeneinstellungen kann die Untersuchung durchgeführt werden. Der C-Bogen 11 wird in die gewünschte Position gebracht, und die Aufnahmen werden entsprechend den festgelegten Parametern ausgelöst.

Der beschriebene Workflow ist lediglich beispielhaft und ermöglicht eine effiziente und sichere Nutzung eines C-Bogen-Röntgengeräts 11. Alle anderen vorstellbaren und zielführenden Workflows sind ebenso vorstellbar und erfindungsgemäß.

### Bezugszeichenliste

- 1: Sensor
- 2: Optionale Tischsensoren
- 3: Optionale Sensoren am Generator
- 4: Patient/Objekt
- 5: Optionale Sensoren am C-Bogen
- 11: C-Bogen
- 12: Röntgenbilddetektor
- 13: Röntgengenerator
- 16, 17: Bildausgabeeinheit
- 19: Eingabeeinheit
- 121: Bildverarbeitungseinheit
- 122: Speichereinheit
- 123: Recheneinheit
- 124: Netzwerkschnittstelle

## Patentansprüche

1. Medizinisches Bildgebungssystem, vorzugsweise ein Röntgengerät, besonders bevorzugt ein C-Bogen, das dazu konfiguriert ist, die Sicherheit von Patienten während eines bildgebenden Verfahrens zu verbessern, wobei das System umfasst:
a. ein C-Bogen-Röntgengerät zur Erzeugung von Röntgenstrahlung, das in der Lage ist, in verschiedene Positionen bewegt zu werden, um unterschiedliche Ansichten des Patienten zu ermöglichen;
b. eine Liege oder Patientenauflage, die dazu konfiguriert ist, den Patienten während des bildgebenden Verfahrens zu positionieren;
c. einen Abstandssensor, der dazu konfiguriert ist, den Abstand zwischen dem C-Bogen-Röntgengerät und/oder Generator und/oder C-Bogen-Gehäuse und/oder dem Patienten und/oder der Liege zu messen;
d. eine Dosisregelungseinheit, die dazu konfiguriert ist, die Strahlungsdosis des Röntgenstrahls basierend auf den gemessenen Abständen zwischen dem C-Bogen-Röntgengerät (Fokuspunkt) und dem Patienten zu regeln; und
e. ein Hautschutzsystem, das dazu konfiguriert ist, in kritischen Situationen die Strahlungsdosis zu unterbrechen und/oder zu minimieren und gleichzeitig eine ausreichende Bildqualität aufrechtzuerhalten, indem es die Strahlungsintensität in Echtzeit anpasst, um die Strahlenexposition der Patientenhaut zu begrenzen.

2. Medizinisches Bildgebungssystem, vorzugsweise ein Röntgengerät, besonders bevorzugt ein C-Bogen, gemäß Anspruch 1, die eine Anzeigeeinrichtung beinhaltet, die dazu konfiguriert ist, visuelle Informationen über den Abstand, die Dosisregelung und den Hautschutz während des bildgebenden Verfahrens anzuzeigen.

3. Medizinisches Bildgebungssystem, vorzugsweise ein Röntgengerät, besonders bevorzugt ein C-Bogen, gemäß einem der vorhergehenden Ansprüche, wobei das System umfasst:
- einen kapazitiven Sensor, der in der Nähe des Generators, des C-Bogens selbst oder des Flatpanelgehäuses positioniert ist, um Änderungen in der Kapazität zwischen dem Sensor und einem Zielobjekt zu erfassen;
- eine Signalauswertungseinheit, die dazu konfiguriert ist, die erfassten Kapazitätsänderungen in Abstandswerte umzuwandeln; und
- eine Anzeigeeinrichtung, die die gemessenen Abstandswerte in einer für medizinisches Personal lesbaren Form ausgibt.

4. Medizinisches Bildgebungssystem, vorzugsweise ein Röntgengerät, besonders bevorzugt ein C-Bogen, gemäß einem der vorhergehenden Ansprüche, wobei das System umfasst:
eine Steuereinheit, die dazu konfiguriert ist, die gemessenen Abstandsinformationen zu analysieren und Kollisionsgefahren zwischen dem C-Bogen-Röntgengerät und dem Patienten und/oder der Liege zu erkennen.

5. Medizinisches Bildgebungssystem, vorzugsweise ein Röntgengerät, besonders bevorzugt ein C-Bogen, gemäß einem der vorhergehenden Ansprüche, wobei das Sensorsystem für den kollisionsschutzbezogenen Einsatz umfasst:
a. einen Sensor zur Erfassung der Entfernung von Objekten in der Nähe des Systems;
b. eine Steuereinheit, die dazu konfiguriert ist, die erfassten Abstände kontinuierlich zu analysieren und Muster oder Trends in den Daten zu identifizieren;
c. eine Algorithmen-Einheit, die dazu konfiguriert ist, die identifizierten Muster oder Trends in den Daten mit vordefinierten Kollisionskriterien zu vergleichen; und
d. eine Reaktionsmechanismus-Einheit, die basierend auf dem Ergebnis des Vergleichs in (c) dazu konfiguriert ist, kollisionsschutzbezogene Maßnahmen zu initiieren, wenn die Kollisionskriterien erfüllt sind.

6. Medizinisches Bildgebungssystem, vorzugsweise ein Röntgengerät, besonders bevorzugt ein C-Bogen gemäß Anspruch 4 und/oder 5, wobei die kollisionsschutzbezogenen Maßnahmen mindestens eine der folgenden Operationen umfassen:
a) eine Warnung an den Bediener des Systems ausgeben;
b) eine Verlangsamung oder Stopp der Bewegung des Systems initiieren;
c) ein automatisches Ausweichmanöver ausführen, um eine Kollision mit einem erkannten Objekt zu verhindern.

7. Medizinisches Bildgebungssystem, vorzugsweise ein Röntgengerät, besonders bevorzugt ein C-Bogen gemäß einem der vorhergehenden Ansprüche, wobei die kollisionsschutzbezogenen Maßnahmen mindestens in alle Richtungen wirken und die hautschutzbezogenen Maßnahmen in Strahlungsrichtung wirken.

8. Medizinisches Bildgebungssystem, vorzugsweise ein Röntgengerät, besonders bevorzugt ein C-Bogen, gemäß einem der vorhergehenden Ansprüche, wobei zusätzlich ein System zur Darstellung von sensorischem Kollisionsschutz und Dosisanpassung auf einem Display vorhanden ist.

9. Medizinisches Bildgebungssystem, vorzugsweise ein Röntgengerät, besonders bevorzugt ein C-Bogen gemäß einem der vorhergehenden Ansprüche, wobei eine Farbcodierungseinheit oder vergleichbare optische- und/oder haptische- und/oder akustische-Codierungseinheiten oder deren Kombination vorhanden ist, die dazu konfiguriert ist, die auf dem Display dargestellten Informationen zu codieren, wobei bestimmte Codes bestimmten Dosisbereichen oder Kollisionsszenarien zugeordnet sind.

10. Medizinisches Bildgebungssystem, vorzugsweise ein Röntgengerät, besonders bevorzugt ein C-Bogen gemäß einem der vorhergehenden Ansprüche, wobei die Überwachung der C-Bogen Positionen über externe oder interne Kameras erfolgt.
